# EUROPEAN PATENT APPLICATION

(11) **EP 1 537 857 A1**
(43) Date of publication of application: **08.06.2005**
(21) Application number: 04101358.2
(22) Date of filing: 01.04.2004
(51) Int. Cl.: A61K 9/06, A61K 31/12, A61K 31/215

(54) **Mupirocin compositions for topical use, a process of making same and uses thereof**

(30) Priority: 03.12.2003 US 526281 P; 03.12.2003 US 526282 P; 08.03.2004 US 794184; 08.03.2004 US 795116
(71) Applicant: AGIS INDUSTRIES (1983) LTD, Bnei-Brak 51200 (IL)
(72) Inventor: Asculai, Eilon, 85338 Lehavim (IL); Zeevi, Amira, 84965 Omer (IL); Lavon, Ilana, 85338 Lehavim (IL); Huri, Rahamim, 84228 Beer-Sheva (IL)
(74) Representative: Schmitz, Jean-Marie

(57) **Abstract**

Spreadable pharmaceutical compositions containing hard fat and Mupirocin, an improved process of preparing same and methods of using same are provided.

## Description

### FIELD AND BACKGROUND OF THE INVENTION

The present invention relates to spreadable pharmaceutical compositions for topical application containing Mupirocin and/or derivatives, a process of manufacturing such compositions, and uses of such compositions.

Mupirocin (pseudomonic acid) is an antibiotic produced by aerobically-cultured *Pseudomonas Fluorescens.* Mupirocin, in the form of free acid, salts and esters, is described in UK Patent No. 1,395,907. Mupirocin and derivatives thereof are mainly effective against gram-positive aerobes and some gram-negative aerobes and are therefore utilized in treating skin, ear and eye infections.

Mupirocin is insoluble in many well-known pharmaceutically acceptable carriers such as glycerol, mineral oil, caprylic-capric triglyceride, isopropyl myristate, isopropyl palmitate, octyl dodecanol (Eutanol G) and isopropyl stearate. Mupirocin is also an unstable molecule easily undergoing hydrolysis and rearrangement reactions in the presence of water, acids and bases (Clayton J.C.S. Perkin 1979: 838-846). The challenge in providing a stable Mupirocin pharmaceutical composition is therefore widely recognizable in the art.

As of this writing, three Mupirocin-containing products are commercially available in the United States: Bactroban® Ointment and Bactroban® Nasal Ointment and Bactroban® Cream, all manufactured by Glaxo SmithKline (Middlesex, UK).

Bactroban® Nasal comprises crystalline Mupirocin calcium dihydrate in a viscous salve carrier, the primary component of the salve being white petrolatum. The petrolatum acts as a waterproof matrix, preventing Mupirocin decomposition. An advantage of this composition is that hydrocarbon-type salves such as those comprising petrolatum are generally occlusive, forming a layer when applied to the skin that reduces water loss and thus increases the moisture content of the skin. On the other hand, hydrocarbon-type salves have an unpleasant greasy feel. Such products are difficult to wash-off and attract dirt. In order to achieve a homogenous thin layer, such products must be rubbed over an area making the use of such a salve unsuitable for use on wounded or otherwise sensitive areas.

In U.S. patent 6,025,389 are taught topical compositions comprising an effective amount of crystalline Mupirocin calcium dehydrate together with mineral oil, between 4 % and 8 % by weight polyoxyethylene ether or ester (e.g., polyethylene glycols monocetyl ether), fatty alcohols or fatty esters and water. Such products have only a limited occlusive effect. Bactroban® Cream is a product based on the teachings of U.S. patent 6,025,389.

In U.S. 4,524,075 are taught topical ointment compositions comprising an anti-bacterially effective amount of Mupirocin, a pharmaceutically acceptable salt thereof or a pharmaceutically acceptable ester thereof and a stabilizing amount of at least 1 % by weight of a poly(alkylene)glycol, a poly(alkoxy substituted alkylene)glycol, a pharmaceutically acceptable ether thereof, a pharmaceutically acceptable ester thereof or a mixture thereof. Therein, the poly(alkylene)glycol or poly(alkoxy substituted alkylene)glycol component acts to stabilize the Mupirocin. Bactroban® Ointment, a product based on the teachings of U.S. patent 4,524,075, comprises a mixture of PEG 400 and PEG 4000.

Ointments based on poly(alkylene)glycols, poly(alkoxy substituted alkylene)glycols and derivatives thereof (collectively referred to as PAG or PAGs) suffer from several drawbacks that limit the use and consumer acceptance of such ointments.

Topically administered PAGs may cause a stinging feeling, urticaria and delayed allergic reactions (see, *The Handbook of Pharmaceutical Excipients 3*^{*rd*} *ed.* 2002 p. 396). The most serious adverse effects associated with PAGs are hyperosmolarity, metabolic acidosis, and renal failure following the topical use by bum patients ("Topical PEG in bum ointment" FDA Drug Bull. 1982, 12: 25). Therefore, despite promising results recorded when using an ointment comprising Mupirocin and PEG to treat burn wounds in rats, PEG containing topical preparations are contraindicated for patients with renal failure, extensive bums, open wounds and damaged skin (Rode *et al.* J. Antimicrob. Chemother. 1988, 21: 589-595; Kaczmarski J. Antimicrob. Chemother. 1988, 22: 771-776; and Rode *et al.* J. Antimicrob. Chemother, 1989, 24: 78-79). As a result, pharmaceutical compositions containing high percentages of PAGs are required to carry a warning label. Products containing none or only low percentages of PAGs are not so labeled.

A further disadvantage of compositions comprising PAGs is that when applied to the skin, these compositions leave an unpleasant sticky feeling on an applied area, reducing patient compliance.

An even further disadvantage of the above-mentioned prior art compositions is that they cannot be applied to mucous membranes.

In the art, hard fats are known as being nontoxic, nonirritating, can be formulated to melt on contact with skin, are compatible with a variety of active pharmaceutical ingredient, and have been approved for pharmaceutical uses. Hard fats have a substantially solid (butter-like) consistency at room temperature. Thus, hard fats are well known for use in the preparation of rectal and vaginal suppositories (see The Science and Practice of Pharmacy, Remington, 19^{th} Ed. pp. 1592). However, hard fats are not generally known for use in the preparation of ointments for topical application.

An exception is found in U.S. Patent No. 6,013,657 where a composition comprising a therapeutically effective amount of Mupirocin, in a carrier comprising a hard fat together with oleyl alcohol and/or castor oil having a smearable consistency is taught. The composition of U.S. Patent No. 6,013,657 has numerous advantages including non-toxicity; suitablity for application to eyes, mucous membranes, open wounds and bum wounds; skin compatibility and spreadability; and superior skin penetration properties (Hermsdorf Cosmetic and toiletries 1980, 95: 61-63).

While practicing the teachings of U.S. Patent No. 6,013,657, the present inventors have uncovered an improved process for preparing the composition disclosed therein. The novel process is characterized by high batch-to-batch viscosity reproducibility and the resulting composition is characterized by physical properties that are highly suitable for ointment preparations and is devoid of the limitations described above.

### SUMMARY OF THE INVENTION

According to the teachings of the present invention there is provided a process of preparing a spreadable pharmaceutical composition for topical application, the process comprising: providing a mixture including Mupirocin, a Mupirocin derivative and/or a Mupirocin pharmaceutically acceptable salt and at least one hard fat; heating the mixture to a first temperature, the first temperature being higher than a congelation temperature of the mixture; cooling the mixture to a second temperature, (preferably to lower than the congellation temperature of the mixture); re-heating the mixture to a third temperature, the third temperature being higher than the congelation temperature of the mixture; and re-cooling the mixture to ambient temperature, thereby obtaining the spreadable pharmaceutical composition of the present invention.

Preferably, the heating, the cooling and the re-heating are performed while the mixture is stirred.

In one embodiment of the present invention the first temperature ranges between about 40 °C and about 100 °C. In another embodiment of the present invention, the first temperature ranges between about 50 °C and about 80 °C.

In one embodiment of the present invention, the second temperature is lower than the congelation temperature of the mixture. In another embodiment of the present invention, the second temperature ranges between about 10 °C and about 30 °C. In a still another embodiment of the present invention, the second temperature ranges between about 18 °C and about 28 °C.

In one embodiment of the present invention, the third temperature ranges between about 30 °C and about 40 °C. In another embodiment of the present invention, the third temperature ranges between about 30 °C and about 35 °C.

In one embodiment of the present invention, the mixture is maintained at the third temperature for a period of time that ranges between about 180 minutes and about 600 minutes. In another embodiment of the present invention, the mixture is maintained at the third temperature for about 300 minutes.

In one embodiment of the present invention, subsequent to the re-heating and prior to the re-cooling, a receptacle or container is filled with the mixture. According to a feature of the embodiment, during the filling the temperature of the mixture is substantially maintained at a fourth temperature. According to one embodiment of the feature of the present invention, the fourth temperature ranges between about 20 °C and about 40 °C. According to another embodiment of the present invention, the fourth temperature ranges between about 30 °C and about 35 °C.

According to a preferred embodiment of the present invention, the mixture further comprises a pharmaceutically acceptable carrier.

According to a feature of the present invention, the pharmaceutically acceptable carrier comprises castor oil and/or oleyl alcohol.

According to a feature of the present invention, the mixture further comprises at least one polyalkyleneglycol (PAG), such as polyethyleneglycol (PEG). According to a feature of the present invention the concentration of the PAG is less than about 20 weight percentages, less than 10 weight percentages or even less than 1 weight percentage of the total weight of the composition. According to a different feature of the present invention, the spreadable pharmaceutical composition is substantially devoid of PAG.

According to a feature of the present invention, the concentration of the at least one hard fat ranges between about 20 weight percentages and about 99 weight percentages of the total weight of the composition. Preferably, the concentration of the at least one hard fat ranges between about 20 weight percentages and about 80 weight percentages of the total weight of the composition.

In one embodiment of the present invention, the at least one hard fat comprises a triglyceride, preferably a mixed triester of glycerin with caprylic, capric and stearic acids.

According to a feature of the present invention, the mixture further comprises a solvent. Suitable solvents include but are not limited to ethanol, n-propanol and isopropanol and mixtures thereof.

According to a feature of the present invention, the mixture further comprises at least one additional component selected from the group consisting of an anti-irritant, an anti-oxidant, a buffering agent, a bulking agent, a colorant, a deodorant, a dermatological active ingredient, a diluent, an emollient, a humectant, a moisturizer, an occlusive agent, oil, a penetration enhancer, a skin penetration enhancer, a perfuming agent, a pH adjusting agent, a preservative, a protectant, a softener, a solubilizer, a stearically stabilizing substance, a sunscreening agent, a sunless tanning agent, a surfactant and a vitamin.

According to a feature of the present invention, the additional component is a surfactant, preferably propylene glycol stearate.

According to a feature of the present invention, the spreadable pharmaceutical composition has a viscosity that ranges between about 2 Poise and about 2500 Poise, preferably between about 2 Poise and about 1500 Poise, at a temperature of about 20 °C.

According to a feature of the present invention, the spreadable pharmaceutical composition is occlusive, creamy, non-greasy, smooth and easy to spread.

According to the teachings of the present invention there is also provided a spreadable pharmaceutical composition, preferably substantially prepared according to the process described hereinabove.

According to a feature of the present invention, the spreadable pharmaceutical composition is substantially devoid of PAG, e.g. PEG.

According to a feature of the present invention, the spreadable pharmaceutical composition is packaged in a packaging material and identified in print, in or on the packaging material, for use for a need selected from the group consisting of curing a condition, treating a condition, preventing a condition, treating symptoms of a condition, curing symptoms of a condition, ameliorating symptoms of a condition, treating effects of a condition, ameliorating effects of a condition, and preventing results of a condition in which treatment by Mupirocin is beneficial.

According to a feature of the present invention the condition is selected from the group consisting of a skin condition, an eye condition, an ear condition, a bum wound, a wound, impetigo, an infection, a fungal infection, a gram-positive aerobe infection, a gram-negative aerobe infection a rectal infection, a vaginal infection and a wound of a mucosal membrane.

According to a feature of the present invention, the composition comprises Mupirocin (as a free acid).

In one embodiment of the present invention, the spreadable pharmaceutical composition further comprises a pharmaceutically acceptable carrier.

According to a feature of the present invention, the pharmaceutically acceptable carrier comprises castor oil and/or oleyl alcohol.

According to a feature of the present invention, the spreadable pharmaceutical composition further comprises at least one polyalkyleneglycol (PAG), such as polyethyleneglycol (PEG). According to a feature of the present invention the concentration of the PAG is less than about 20 weight percentages, less than 10 weight percentages or even less than 1 weight percentage of the total weight of the composition. According to a different feature of the present invention, the spreadable pharmaceutical composition is substantially devoid of PAG.

According to a feature of the present invention, the concentration of the at least one hard fat ranges between about 20 weight percentages and about 99 weight percentages of the total weight of the spreadable pharmaceutical composition. According to a different feature of the present invention, the concentration of the at least one hard fat ranges between about 20 weight percentages and about 80 weight percentages of the total weight of the spreadable pharmaceutical composition.

In one embodiment of the present invention, the at least one hard fat comprises a triglyceride, preferably a mixed triester of glycerin with caprylic, capric and stearic acids.

According to a feature of the present invention, the spreadable pharmaceutical composition further comprises a solvent. Suitable solvents include but are not limited to ethanol, n-propanol and isopropanol and mixtures thereof.

According to a feature of the present invention, the spreadable pharmaceutical composition further comprises at least one additional component selected from the group consisting of an anti-irritant, an anti-oxidant, a buffering agent, a bulking agent, a colorant, a deodorant, a dermatological active ingredient, a diluent, an emollient, a humectant, a moisturizer, an occlusive agent, oil, a penetration enhancer, a skin penetration enhancer, a perfuming agent, a pH adjusting agent, a preservative, a protectant, a softener, a solubilizer, a stearically stabilizing substance, a sun screening agent, a sunless tanning agent, a surfactant and a vitamin.

According to a feature of the present invention, the spreadable pharmaceutical composition further comprises propylene glycol stearate as a surfactant.

According to a feature of the present invention, the spreadable pharmaceutical composition has a viscosity that ranges between about 2 Poise and about 2500 Poise, preferably between about 2 Poise and about 1500 Poise, at a temperature of about 20 °C.

According to a feature of the present invention, the spreadable pharmaceutical composition is occlusive, creamy, non-greasy, smooth and easy to spread.

According to the teachings of the present invention there is also provided a method of treatment comprising administering an effective amount of the spreadable pharmaceutical composition of the present invention, described hereinabove, to a mammal in need thereof.

According to a feature of the method of the present invention the mammal is either a non-human mammal or a human.

According to feature of the method of the present invention the spreadable pharmaceutical composition is administered by topically applying to ears, eyes, skin and/or mucous membranes.

According to another feature of the method of the present invention the spreadable pharmaceutical composition is administered buccally, dermally, intranasally, lingually, mucosally, rectally, sublingually, topically, urethrally and vaginally.

According to feature of the method of the present invention the need for which the spreadable pharmaceutical composition is applied arises from a medical condition selected from the group consisting of a skin condition, an eye condition, an ear condition, a burn wound, a wound, impetigo, an infection, a fungal infection, a gram-positive aerobe infection, a gram-negative aerobe infection, a rectal infection, a vaginal infection and a wound of a mucosal membrane.

According to feature of the method of the present invention the need for which the spreadable pharmaceutical composition is applied is selected from the group consisting of curing the condition, treating the condition, preventing the condition, treating symptoms of the condition, curing symptoms of the condition, ameliorating symptoms of the condition, treating effects of the condition, ameliorating effects of the condition, and preventing results of the condition.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

### DESCRIPTION OF THE PREFERRED EMBODIMENTS

The present invention is of a process for making a spreadable pharmaceutical composition that is substantially a Mupirocin-containing composition based on hard fats, in which a spreadable pharmaceutical composition characterized by high suitability for topical application is obtained. The present invention is therefore also of spreadable pharmaceutical compositions, and particularly of a spreadable pharmaceutical composition made according to the process of the present invention, and of a method of treatment using the spreadable pharmaceutical composition.

The principles and uses of the processes, compositions and methods of the present invention may be better understood with reference to the description and examples hereinbelow.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

As discussed in the introduction hereinabove, the susceptibility of Mupirocin to decomposition requires its formulation in the presence of a waterless carrier, preferably a hydrophobic carrier. Therefore emulsions such as creams and lotions are usually inappropriate as carriers for Mupirocin, whereas ointments are presently considered as preferred carriers in this respect. As is discussed in detail hereinabove, the presently known Mupirocin preparations are petrolatum and high PEG-content based ointments, which have an unpleasant "skin-feel". Further, petrolatum ointments are difficult to spread while high PEG-content ointments are often contraindicated for conditions where Mupirocin compositions are desired. In the art it is known that it is difficult to provide an ointment comprising Mupirocin as an active ingredient in a PEG-free or low-PEG carrier.

The present inventors have now surprisingly uncovered a novel process in which a spreadable composition comprising hard fat and Mupirocin (including free acid Mupirocin, a Mupirocin derivative and/or a Mupirocin pharmaceutically acceptable salt) as an active ingredient, which is highly suitable for topical application is obtained. The process of the present invention reproducibly produces a composition that is creamy, non-greasy, occlusive, smooth and easy to spread, melts on the skin and needs not be rubbed over an area to achieve a homogenously thin layer.

As used herein, the term "spreadable" is used to mean that the composition can be easily spread over an area.

The term "creamy" is used herein to mean that the composition has application characteristics similar to a cream.

The term "occlusive" is used to mean that the composition occludes the area onto which it is applied.

The term "non-greasy" is used to mean that the composition is devoid of greasiness, or, in other words, that no greasy feeling accompanies the application of the composition.

The term "smooth" is used to mean that the composition is characterized by a "creamy like" feeling when applied.

The phrase "easy to spread" is used to mean that the composition needs not be rubbed over an area to achieve a homogenously thin layer.

The properties described hereinabove are mainly attributed to the unique property of hard fat based compositions, which tend to melt upon contacting a body area.

As used herein, the term "active ingredient" is a component of a composition that has a pharmaceutical activity.

As used herein the term "topical application" describes application onto a biological surface, e.g., an affected skin or membrane. Hence, the phrase "a composition for topical application" describes a composition that is applied, preferably by spreading, on, for example, wounds, bums and an affected skin, eye, ear or membrane of a subject.

As used herein, the term "process" and the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

The process of the present invention for preparing a spreadable pharmaceutical composition of the present invention comprises: providing a mixture (comprising Mupirocin, a Mupirocin derivative and/or a Mupirocin pharmaceutically acceptable salt and at least one hard fat); heating the mixture to a first temperature, which is higher than a congelation temperature of the mixture; cooling the mixture to a second temperature which is preferably lower than a congelation temperature of the mixture; re-heating the mixture to a third temperature, which is higher than the congelation temperature of the mixture; and re-cooling the mixture to ambient temperature, thereby obtaining the spreadable pharmaceutical composition for topical application. Preferably, the heating, the cooling and the re-heating are performed while the mixture is stirred.

As used herein, the term "congelation temperature" refers to the highest temperature at which the mixture congeals, that is that the mixture starts to become solid rather than liquid.

According to a feature of the present invention, providing the mixture comprises mixing the at least one hard fat and Mupirocin.

The term "Mupirocin" encompasses herein mupirocin as a free acid, as a pharmaceutically acceptable salt thereof or any derivative thereof, unless otherwise indicated. Preferably, the Mupirocin used in the process of the present invention is a free acid Mupirocin, which is also referred to herein simply as Mupirocin.

The concentration of the Mupirocin preferably ranges between about 0.5 weight percentage and about 5 weight percentages, more preferably between about 1 weight percentage and about 5 weight percentages, more preferably between about 1 weight percentage and about 3 weight percentages, and most preferably it is about 2 weight percentages of the total weight of the composition.

By cooling or re-cooling is meant herein that the temperature of the mixture is reduced either actively (by cooling) or passively (by letting the mixture cool down). Preferably the mixture is mixed during the cooling process.

According to a preferred embodiment, the first temperature ranges between about 30 °C and about 100 °C, preferably between about 40 °C and about 100 °C, more preferably between about 40 °C and about 90 °C, more preferably between about 50 °C and about 90 °C, more preferably between about 50 °C and about 80 °C, and even more preferably between about 60 °C and about 80 °C.

As used herein, the term "about" refers to ± 10 %.

According to another preferred embodiment, during the heating step, the mixture is maintained at the first temperature for a time period that ranges between about 10 minutes and about 240 minutes, more preferably between about 15 minutes and about 120 minutes, more preferably between about 15 minutes and about 60 minutes.

After the heating step, the mixture is cooled to a second temperature that is lower than a congelation temperature of the mixture.

As a typical congelation temperature of the hard fat-based mixture of the present invention is lower than 30 °C, according to a preferred embodiment, the second temperature ranges between about 10 °C and about 30 °C, more preferably between about 15 °C and about 30 °C, more preferably between about 18 °C and about 28 °C, and most preferably between about 20 °C and about 25 °C.

Once the mixture reaches the second temperature, it is re-heated to a third temperature, which again, is higher than the congelation temperature of the mixture. According to a preferred embodiment, the third temperature ranges between about 30 °C and about 40 °C, more preferably between about 30 °C and about 35 °C.

In a preferred embodiment, the mixture is maintained at the third temperature for a period of time that ranges between about 180 minutes and about 600 minutes, more preferably between about 240 minutes and about 480 minutes, preferably about 300 minutes.

In another preferred embodiment of the present invention, subsequent to re-heating and prior to re-cooling the mixture, a receptacle is filled with the mixture. Preferably, during the filling of the receptacle, the temperature of the mixture is substantially maintained at a fourth temperature that ranges between about 20 °C and about 40 °C, or more preferably between about 30 °C and about 35 °C.

The process of the present invention is preferably performed in greater than laboratory scale and has certain advantages when so performed. Thus the process of the present invention is preferably performed when the volume of the mixture is greater than 1 liter, more preferably greater than 2 liter, even more preferably greater than 3 liter, even more preferably greater than 4 liter, even more preferably greater than 5 liter, even more preferably greater than 6 liter, even more preferably greater than 7 liter, even more preferably greater than 8 liter, even more preferably greater than 9 liter and even more preferably greater than 10 liter.

The receptacle can be, for example, a tube (such as a squeeze tube), a jar or a bottle (for example with a pump dispenser).

According to a feature of the present invention, the spreadable pharmaceutical composition thus produced has a viscosity that ranges between about 2 Poise and about 2500 Poise at a temperature of about 20 °C, more preferably between about 2 Poise and about 1500 Poise. As is demonstrated in the Examples section that follows, the viscosity of the composition remains substantially unchanged for at least 6 months.

The advantageous properties of the spreadable pharmaceutical composition produced by the process of the present invention are mainly attributed to the presence of the hard fat(s). Without being bound to any particular theory, it is assumed that these properties are obtained by re-altering the mixture temperature as described hereinabove.

The term "hard fat" is used herein as understood in the United States Pharmacopoeia and substantially as defined in The Handbook of Pharmaceutical Excipients, 3^{rd} Edition, page 550. As described therein, hard fats typically consist mainly of mixtures of the triglyceride esters of the higher saturated fatty acids (C₈H₁₇COOH through C₁₈H₃₇COOH). Hard fats optionally include varying proportions of mono- and diglycerides. For the present invention the term "hard fat" also includes substantially pure triglyceride esters.. A preferred hard fat comprises a triglyceride, preferably a mixed triester of glycerin with caprylic, capric and stearic acids.

According to a feature of the present invention, the at least one hard fat preferably ranges between about 20 weight percentages and about 99 weight percentages, more preferably between about 20 weight percentages and about 80 weight percentages, even more preferably between about 60 weight percentages and about 80 weight percentages, of the total weight of said composition.

According to another feature of the present invention, the mixture and the resulting composition further comprise a pharmaceutically acceptable carrier.

As used herein, the term "pharmaceutically acceptable carrier" describes a carrier or a diluent that does not cause significant irritation to an organism and does not abrogate the biological activity and properties of the applied active ingredient.

As described in U.S. patent 6,013,657, it is advantageous to add oleyl alcohol, castor oil, and mixtures thereof as carriers.

Other suitable carriers include, for example, water, liquid alcohols, liquid glycols, liquid polyalkylene glycols, liquid esters, liquid amides, liquid protein hydrolysates, liquid alkylated protein hydrolysates, liquid lanolin and lanolin derivatives, and like materials commonly employed in cosmetic and medicinal compositions. Other suitable carriers herein include for example alcohols, including both monohydric and polyhydric alcohols, e.g., ethanol, isopropanol, glycerol, sorbitol, 2-methoxyethanol, diethyleneglycol, ethylene glycol, hexyleneglycol, mannitol, and propylene glycol; ethers such as diethyl or dipropyl ether; polyethylene glycols and methoxypolyoxyethylenes (carbowaxes having molecular weight ranging from 200 to 20,000); polyoxyethylene glycerols, polyoxyethylene sorbitols, stearoyl diacetin, and the like.

The concentration of a pharmaceutically acceptable carrier according to the present invention preferably ranges between about 10 weight percentages and about 80 weight percentages, more preferably between about 10 weight percentages and about 60 weight percentages, more preferably between about 10 weight percentages and about 40 weight percentages, more preferably between about 10 weight percentages and about 30 weight percentages, and most preferably it is about 20 weight percentages of the total weight of the composition.

According to a feature of the present invention, the mixture may further comprise at least one PAG, as is detailed hereinabove. In one embodiment of the present invention, the concentration of PAG is less than about 20, preferably less than about 10, more preferably less than about 1 weight percentages of the total weight of the composition. Due to the disadvantages of using PAG in compositions as described in the Background section above, it is preferred that the mixture be substantially devoid of PAG, particularly devoid of PEG.

As used herein, the phrase "substantially devoid of PAG" means that a composition does not include PAG or include a minor concentration of PAG, which does not cause any of the adverse side effects associated with PAG application described hereinabove. Such a concentration typically ranges between about 0.1 and 20 weight percentages, preferably between about 0.1 and 10 weight percentages, more preferably between about 0.1 and 5 weight percentages and more preferably between about 0.1 and 1 weight percentages of the total weight of the composition.

According to a feature of the present invention, the mixture may further comprise a solvent. Suitable solvents that can be used in preparing a composition of the present invention include, but are not limited to, ethanol, n-propanol, isopropanol and mixtures thereof.

In a preferred embodiment, the process of the present invention also includes adding to the mixture an additional component or additional components that are suitable for rendering the composition of the present invention more cosmetically or aesthetically acceptable or to provide the composition with additional usage benefits. The addition can be done at any stage although it is generally most simple to add additional components before or during the heating of the mixture.

Additional components include any pharmaceutically acceptable or cosmetically acceptable ingredients such as those found in the CTFA International Cosmetic Ingredient Dictionary and Handbook, 8th edition, edited by Wenninger and Canterbery (The Cosmetic, Toiletry, and Fragrance Association, Inc., Washington, D.C., 2000). Representative examples include but are not limited to anti-irritants, anti-oxidants, buffering agents, bulking agents, colorants, deodorants, dermatological active ingredients, diluents, emollients, humectants, moisturizers, occlusive agents, penetration enhancers and skin penetration enhancers, perfuming agents, pH adjusting agents, preservatives, protectants, softeners, solubilizers, stearically stabilizing substances, sun screening agents, sunless tanning agents, surfactants and vitamins.

Suitable anti-irritants that can be used in preparing a composition of the present invention include, but are not limited to, steroidal and non steroidal antiinflammatory agents or other materials such as aloe vera, chamomile, alpha-bisabolol, cola nitida extract, green tea extract, tea tree oil, licoric extract, allantoin, caffeine or other xanthines, glycyrrhizic acid and its derivatives.

Suitable anti-oxidants that can be used in preparing a composition of the present invention include, but are not limited to, ascorbic acid (vitamin C) and its salts, ascorbyl esters of fatty acids, ascorbic acid derivatives (e.g., magnesium ascorbyl phosphate, sodium ascorbyl phosphate, ascorbyl sorbate), tocopherol (vitamin E), tocopherol sorbate, tocopherol acetate, other esters of tocopherol, butylated hydroxy benzoic acids and their salts, 6-hydroxy-2,5,7,8-tetramethylchroman-2-carboxylic acid (commercially available under the tradename Trolox®), gallic acid and its alkyl esters, especially propyl gallate, uric acid and its salts and alkyl esters, sorbic acid and its salts, lipoic acid, amines (e.g., N,N-diethylhydroxylamine, amino-guanidine), sulfhydryl compounds (e.g., glutathione), dihydroxy fumaric acid and its salts, lycine pidolate, arginine pilolate, nordihydroguaiaretic acid, bioflavonoids, curcumin, lysine, methionine, proline, superoxide dismutase, silymarin, tea extracts, grape skin/seed extracts, melanin, and rosemary extracts.

Suitable dermatological inactive ingredients that can be used in preparing a composition of the present invention include, but are not limited to, jojoba oil and aromatic oils such as methyl salicylate, wintergreen, peppermint oil, bay oil, eucalyptus oil and citrus oils, as well as ammonium phenolsulfonate, bismuth subgallate, zinc phenolsulfonate and zinc salicylate.

Suitable emollients that can be used in preparing a composition of the present invention include, but are not limited to, ammonium lactate, dodecane, squalane, cholesterol, isohexadecane, isononyl isononanoate, PPG Ethers, petrolatum, lanolin, safflower oil, castor oil, coconut oil, cottonseed oil, palm kernel oil, palm oil, peanut oil, soybean oil, polyol carboxylic acid esters, derivatives thereof and mixtures thereof.

Suitable humectants and moisturizers that can be used in preparing a composition of the present invention include, but are not limited to ammonium lactate, urea, guanidine, glycolic acid and glycolate salts (*e.g.* ammonium salt and quaternary alkyl ammonium salt), aloe vera in any of its variety of forms (*e.g.*, aloe vera gel), allantoin, urazole, polyhydroxy alcohols such as sorbitol, glycerol, hexanetriol, propylene glycol, butylene glycol, hexylene glycol and the like, polyethylene glycols, sugars and starches, sugar and starch derivatives (*e.g.*, alkoxylated glucose), hyaluronic acid, lactamide monoethanolamine, acetamide monoethanolamine and any combination thereof.

Suitable occlusive agents that can be used in preparing a composition of the present invention include, but are not limited to, petrolatum, mineral oil, beeswax, silicone oil, lanolin and oil-soluble lanolin derivatives, saturated and unsaturated fatty alcohols such as behenyl alcohol, hydrocarbons such as squalane, and various animal and vegetable oils such as almond oil, peanut oil, wheat germ oil, linseed oil, jojoba oil, oil of apricot pits, walnuts, palm nuts, pistachio nuts, sesame seeds, rapeseed, cade oil, corn oil, peach pit oil, poppyseed oil, pine oil, castor oil, soybean oil, avocado oil, safflower oil, coconut oil, hazelnut oil, olive oil, grape seed oil and sunflower seed oil.

Suitable penetration enhancers that can be used in preparing a composition of the present invention include, but are not limited to, dimethylsulfoxide (DMSO), dimethyl formamide (DMF), allantoin, urazole, N,N-dimethylacetamide (DMA), decylmethylsulfoxide (C₁₀ MSO), polyethylene glycol monolaurate (PEGML), propylene glycol (PG), propylene glycol monolaurate (PGML), glycerol monolaurate (GML), lecithin, the 1-substituted azacycloheptan-2-ones, particularly 1-n-dodecylcyclazacycloheptan-2-one, alcohols, and the like. Suitable penetration enhancers include vegetable oils including, for example, safflower oil, cottonseed oil and corn oil.

Suitable pH adjusting agents that can be used in preparing a composition of the present invention include, but are not limited to, one or more adipic acids, glycines, citric acids, calcium hydroxides, magnesium aluminometasilicates, buffers or any combinations thereof.

Suitable preservatives that can be used in preparing a composition of the present invention include, but are not limited to, one or more alkanols, disodium EDTA (ethylenediamine tetraacetate), EDTA salts, EDTA fatty acid conjugates, isothiazolinone, parabens such as methylparaben and propylparaben, propylene glycols, sorbates, urea derivatives such as diazolindinyl urea, or any combinations thereof.

Suitable softeners that can be used in preparing a composition of the present invention include, but are not limited to, ammonium lactate, dodecane, squalane, cholesterol, isohexadecane, isononyl isononanoate, PPG Ethers, petrolatum, lanolin, safflower oil, castor oil, coconut oil, cottonseed oil, palm kernel oil, palm oil, peanut oil, soybean oil, polyol carboxylic acid esters, derivatives thereof and mixtures thereof.

Suitable solubilizers that can be used in preparing a composition of the present invention include, but are not limited to, complex-forming solubilizers such as citric acid, ethylenediamine-tetraacetate, sodium meta-phosphate, succinic acid, urea, cyclodextrin, polyvinylpyrrolidone, diethylammonium-ortho-benzoate, and micelle-forming solubilizers such as TWEENS and spans, *e.g.,* TWEEN 80. Other solubilizers that are usable for the compositions of the present invention are, for example, polyoxyethylene sorbitan fatty acid ester, polyoxyethylene n-alkyl ethers, n-alkyl amine n-oxides, poloxamers, organic solvents, phospholipids and cyclodextrines.

Suitable stearically stabilizing substances that can be used in preparing a composition of the present invention include, but are not limited to, poloxamers and poloxamines (block copolymers of polyoxyethylene and polyoxypropylene), ethoxylated esters of sorbitan fatty acids, in particular polysorbates (such as polysorbate 80 or Tween 80), ethoxylated mono- and diglycerides, ethoxylated lipids, ethoxylated fatty alcohols or fatty acids, and charged ionic stabilizers and peptizing agents, such as diacetyl phosphates, phosphatidyl glycerol, as well as saturated and unsaturated fatty acids, sodium cholate, sodium glycocholate, sodium taurocholate or mixtures thereof, aminoacids or peptizing agents such as sodium citrate (Lucks Int. J. Pharmaceutics 1990 63: 183-188).

Suitable surfactants that can be used in preparing a composition of the present invention include, but are not limited to, ethoxylated esters of sorbitan fatty acids, block polymers and block copolymers (such as poloxamers and poloxamines), polyglycerol ethers and polyglycerol esters, lecithins of various origins (such as egg lecithin or soya lecithin), chemically modified lecithins (such as hydrogenated lecithins), as well as phospholipids and sphingolipids, mixtures of lecithins with phospholipids, sterols (such as cholesterol and its derivatives, specifically stigmasterol), esters and ethers of sugars or of sugar alcohols with fatty acids or fatty alcohols (such as saccharose monostearate).

In a preferred embodiment of the present invention, the mixture and as a result the pharmaceutical composition comprises a surfactant, preferably propylene glycol stearate.

The concentration of the surfactant, or of any of the additional component(s) described above preferably ranges between about 1 weight percentage and about 20 weight percentages, more preferably between about 5 weight percentages and about 15 weight percentages, more preferably between about 5 weight percentages and about 10 weight percentages and most preferably it is about 8 weight percentages of the total weight of the composition.

Suitable vitamins that can be used in preparing a composition of the present invention include, but are not limited to, vitamin A and its analogs and derivatives: retinol, retinal, retinyl palmitate, retinoic acid, tretinoin, iso-tretinoin (known collectively as retinoids), vitamin E (tocopherol and its derivatives), vitamin C (L-ascorbic acid and its esters and other derivatives), vitamin B₃ (niacinamide and its derivatives), alpha hydroxy acids (such as glycolic acid, lactic acid, tartaric acid, malic acid, citric acid, etc.) and beta hydroxy acids (such as salicylic acid and the like).

According to another aspect of the present invention, there is provided a spreadable pharmaceutical composition comprising Mupirocin, derivatives thereof and/or pharmaceutically acceptable salts thereof and one or more hard fat(s).

In a preferred embodiment of the present invention, the pharmaceutical composition is substantially prepared according to the process described hereinabove.

The spreadable pharmaceutical composition of the present invention is preferably packaged in a packaging material and is identified in print, in or on the packaging material, for use for a need selected from the group, including but not limited to curing a condition, treating a condition, preventing a condition, treating symptoms of a condition, curing symptoms of a condition, ameliorating symptoms of a condition, treating effects of a condition, ameliorating effects of a condition, and preventing results of a condition in which treatment by Mupirocin is beneficial.

Mupirocin is known to be beneficial for the treatments of many conditions including but not limited to skin conditions, eye conditions, ear conditions, and conditions of mucous membranes, including wounds, bums, infections (e.g. fungal infections, infections by gram-positive aerobes, impetigo), of the skin, eye, ear, nasal passages, mouth passages, rectum, vagina, urether and other mucosal membranes.

The packaging is preferably performed during the preparation process, before re-cooling the mixture, as is detailed hereinabove.

The package or receptacle in which the compositions of the present invention is packed preferably comprises a tube (such as a squeeze tube), a jar, a bottle (for example with a pump dispenser), a unit dose or a pressurized container, using techniques well known to those skilled in the art and as set forth in reference works such as Remington's Pharmaceutical Science 15^{th} Ed. It is preferred that the package or receptacle is such that, during use, contact of the unused compositions with the environment is minimized.

A spreadable pharmaceutical composition prepared according to the process described hereinabove is used in implementing a method of treatment.

Thus, the present invention also provides for a method of treatment comprising administering a pharmaceutically effective amount of the spreadable pharmaceutical composition of the present invention to a mammal in need thereof. Such a mammal can be a human or a non-human mammal.

Administration of the spreadable pharmaceutical composition is preferably performed by topically applying to ears, eyes, skin and/or mucous membranes. Administration of the spreadable pharmaceutical composition may further be performed buccally, dermally, intranasally, lingually, mucosally, rectally, sublingually, topically, urethrally and vaginally.

The term "pharmaceutically effective amount" describes an amount of a composition that is sufficient to significantly induce a positive modification in the condition being treated, but low enough to avoid significant side effects, within the scope of sound judgment of one skilled in the art. The effective amount of the composition may vary with the particular area being treated, the age and physical condition of the mammal being treated, the severity of the condition, the duration of the treatment, the nature of concurrent therapy, the specific compound, composition or other material employed, the particular carrier utilized, and like factors within the knowledge and expertise of one skilled in the art.

Medical conditions for which the treatment of the present invention is useful are generally those for which Mupirocin is known to be beneficial, as delineated hereinabove.

The need for which the treatment of the present invention is used includes but is not limited to curing the condition, treating the condition, preventing the condition, treating symptoms of the condition, curing symptoms of the condition, ameliorating symptoms of the condition, treating effects of the condition, ameliorating effects of the condition, and preventing results of the condition.

Additional objects, advantages, and novel features of the present invention will become apparent to one ordinarily skilled in the art upon examination of the following non-limiting, example. Additionally, each of the various embodiments and aspects of the present invention as delineated hereinabove and as claimed in the claims section below finds experimental support in the following examples.

### EXAMPLES

Reference is now made to the following examples, which together with the above description illustrate the invention in a non-limiting fashion.

### MATERIALS AND EXPERIMENTAL METHODS

### Materials:

Reagents were purchased from various commercial suppliers.

The hard fat used (caprylic/capric/stearic triglyceride), was purchased from SASOL, under the name Sofistan 378.

### Experimental methods:

Semi-industrial scale syntheses were performed in a Scott Turbon Mixer having a capacity of 20 liters.

Viscosity of compositions was measured using a Brookfield Voscometer RVDV -1+1.

### EXAMPLE 1

### SMALL SCALE PREPARATION OF A MUPIROCIN COMPOSITION

70 grams hard fat (caprylic/capric/stearic triglyceride), 15 grams castor oil, 8 grams propylene glycol stearate, 5 grams oleyl alcohol and 2 grams Mupirocin were placed in a 100 ml glass flask fitted with a thermometer, a mechanical stirrer and immersed in an oil bath. The oil bath was heated to 75 °C and the mixture stirred for 30 minutes. The flask was removed from the oil and the mixture allowed to cool while stirring. The mixture was observed to congeal at a temperature of about 25 °C. The flask containing the mixture was then immersed in a 32 °C oil bath and stirred for five hours. The flask was removed from the bath, the resulting molten mixture was poured in a jar and was allowed to cool to room temperature. The congealed product was labeled product I.

### EXAMPLE 2

### SEMI-INDUSTRIAL PREPARATION OF MUPIROCIN COMPOSITIONS

7000 grams hard fat (caprylic/capric/stearic triglyceride), 1500 grams castor oil, 800 grams propylene glycol stearate, 500 grams oleyl alcohol and 200 grams Mupirocin were placed in a semi-industrial scale reactor. The mixture was heated to 75 °C and was stirred for 30 minutes. The mixture was allowed to cool in the reactor to 25 °C, while stirring. The mixture was stirred at 25 °C for a period of 60 minutes. The mixture was then re-heated to 32 °C oil bath and was stirred for five hours. While stirring, a 15 grams aluminum squeeze-tube was filled with the mixture and was thereafter allowed to cool to room temperature. The above procedure was repeated 4 times. Each of the squeeze-tubes was labeled as containing product II, III, IV or V.

### EXAMPLE 3

### VISCOSITY

After 1 day the viscosities of products I -V were measured and found to be 4 Poise at 20 °C. After 6 months storage at 20 °C, the viscosities of products I-V were measured and found to be unchanged.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention.

## Claims

1. A process of preparing a spreadable pharmaceutical composition for topical application, the process comprising:
providing a mixture including Mupirocin and/or a Mupirocin derivative and/or a Mupirocin pharmaceutically acceptable salt and at least one hard fat;
heating said mixture to a first temperature, said first temperature being higher than a congelation temperature of said mixture;
cooling said mixture to a second temperature;
re-heating said mixture to a third temperature, said third temperature being higher than said congelation temperature of said mixture; and
re-cooling said mixture to ambient temperature, thereby obtaining the spreadable pharmaceutical composition for topical application.

2. The process of claim 1, wherein said second temperature is lower than said congelation temperature of said mixture.

3. The process of claim 1, wherein said mixture comprises free acid Mupirocin.

4. The process of claim 1, wherein a concentration of aid Mupirocin ranges between about 0.5 weight percentage and about 5 weight percentages of the total weight of said composition.

5. The process of claim 1, wherein said heating is performed while stirring said mixture.

6. The process of claim 1, wherein said cooling is performed while stirring said mixture.

7. The process of claim 1, wherein said re-heating is performed while stirring said mixture.

8. The process of claim 1, wherein said first temperature ranges between about 40 °C and about 100 °C.

9. The process of claim 8, wherein said first temperature ranges between about 50 °C and about 80 °C.

10. The process of claim 1, wherein said second temperature ranges between about 10 °C and about 30 °C.

11. The process of claim 10, wherein said second temperature ranges between about 18 °C and about 28 °C.

12. The process of claim 1, wherein said third temperature ranges between about 30 °C and about 40 °C.

13. The process of claim 12, wherein said third temperature ranges between about 30 °C and about 35 °C.

14. The process of claim 1, wherein said mixture is maintained at said third temperature for a period of time that ranges between about 180 minutes and about 600 minutes.

15. The process of claim 14, wherein said mixture is maintained at said third temperature for about 300 minutes.

16. The process of claim 1, further comprising, subsequent to said re-heating and prior to said re-cooling, filling a receptacle with said mixture.

17. The process of claim 16, wherein during said filling a temperature of said mixture is substantially maintained at a fourth temperature.

18. The process of claim 17, wherein said fourth temperature ranges between about 20 °C and about 40 °C.

19. The process of claim 18, wherein said fourth temperature ranges between about 30 °C and about 35 °C.

20. The process of claim 1, wherein said mixture further comprises a pharmaceutically acceptable carrier.

21. The process of claim 20, wherein said pharmaceutically acceptable carrier comprises castor oil and/or oleyl alcohol.

22. The process of claim 1, wherein said mixture further comprises at least one poly(alkylene)glycol (PAG).

23. The process of claim 22, wherein a concentration of said PAG is less than about 20 weight percentages of the total weight of the composition.

24. The process of claim 22, wherein a concentration of said PAG is less than about 10 weight percentages of the total weight of the composition.

25. The process of claim 22, wherein a concentration of said PAG is less than about 1 weight percentages of the total weight of the composition.

26. The process of claim 1, wherein a concentration of said at least one hard fat ranges between about 20 weight percentages and about 99 weight percentages of the total weight of said composition.

27. The process of claim 1, wherein a concentration of said at least one hard fat ranges between about 20 weight percentages and about 80 weight percentages of the total weight of said composition.

28. The process of claim 1, wherein said at least one hard fat comprises a triglyceride.

29. The process of claim 28, wherein said triglyceride is a mixed triester of glycerin with caprylic, capric and stearic acids.

30. The process of claim 1, wherein said mixture further comprises a solvent.

31. The process of claim 30, wherein said solvent is selected from the group consisting of ethanol, n-propanol and isopropanol and mixtures thereof.

32. The process of claim 1, wherein said mixture further comprises at least one additional component selected from the group consisting of an anti-irritant, an anti-oxidant, a buffering agent, a bulking agent, a colorant, a deodorant, a dermatological active ingredient, a diluent, an emollient, a humectant, a moisturizer, an occlusive agent, oil, a penetration enhancer, a skin penetration enhancer, a perfuming agent, a pH adjusting agent, a preservative, a protectant, a softener, a solubilizer, a stearically stabilizing substance, a sun screening agent, a sunless tanning agent, a surfactant and a vitamin.

33. The process of claim 32, wherein said additional component is a surfactant.

34. The process of claim 33, wherein said surfactant is propylene glycol stearate.

35. The process of claim 1, wherein the spreadable pharmaceutical composition is substantially devoid of PAG.

36. The process of claim 1, wherein the spreadable pharmaceutical composition has a viscosity that ranges between about 2 Poise and about 2500 Poise at a temperature of about 20 °C.

37. The process of claim 1, wherein the spreadable pharmaceutical composition has a viscosity that ranges between about 2 Poise and about 1500 Poise at a temperature of about 20 °C.

38. The process of claim 1, wherein said composition is non-greasy, occlusive, creamy, smooth and easy to spread.

39. A spreadable pharmaceutical composition substantially prepared according to the process of claims 1-38.

40. The spreadable pharmaceutical composition of claim 39, packaged in a packaging material and identified in print, in or on said packaging material, for use for a need selected from the group consisting of curing a condition, treating a condition, preventing a condition, treating symptoms of a condition, curing symptoms of a condition, ameliorating symptoms of a condition, treating effects of a condition, ameliorating effects of a condition, and preventing results of a condition in which treatment by Mupirocin is beneficial.

41. The spreadable pharmaceutical composition of claim 40, wherein said condition is selected from the group consisting of a skin condition, an eye condition, an ear condition, a burn wound, a wound, impetigo, an infection, a fungal infection, a gram-positive aerobe infection, a gram-negative aerobe infection a rectal infection, a vaginal infection and a wound of a mucosal membrane.

42. Use of a pharmaceutically effective amount of the spreadable pharmaceutical composition of claims 39-41 for the manufacture of a medicament to be administered to a mammal in need thereof.

43. Use of claim 42, wherein said mammal is a non-human mammal.

44. Use of claim 42, wherein said mammal is a human.

45. Use of claims 42-44, wherein said administering is effected by topically applying said spreadable pharmaceutical composition to an ear, an eye, a skin and/or a mucous membrane.

46. Use of claims 42-45, wherein said administering is effected in a manner selected from the group consisting of buccally, dermally, intranasally, lingually, mucosally, rectally, sublingually, topically, urethrally and vaginally.

47. Use of claim 42-46, wherein said need arises from a condition in which treatment by Mupirocin is beneficial.

48. A spreadable pharmaceutical composition comprising Mupirocin and/or a Mupirocin derivative and/or a Mupirocin pharmaceutically acceptable salt and at least one hard fat.

49. The spreadable pharmaceutical composition of claim 48, packaged in a packaging material and identified in print, in or on said packaging material, for use for a need selected from the group consisting of curing a condition, treating a condition, preventing a condition, treating symptoms of a condition, curing symptoms of a condition, ameliorating symptoms of a condition, treating effects of a condition, ameliorating effects of a condition, and preventing results of a condition in which treatment by Mupirocin is beneficial.

50. The spreadable pharmaceutical composition of claim 49, wherein said condition is selected from the group consisting of a skin condition, an eye condition, an ear condition, a burn wound, a wound, impetigo, an infection, a fungal infection, a gram-positive aerobe infection, a gram-negative aerobe infection a rectal infection, a vaginal infection and a wound of a mucosal membrane.

51. The spreadable pharmaceutical composition of claim 48, further comprising a pharmaceutically acceptable carrier.

52. The spreadable pharmaceutical composition of claim 48, further comprising at least one additional component selected from the group consisting of an anti-irritant, an anti-oxidant, a buffering agent, a bulking agent, a colorant, a deodorant, a dermatological active ingredient, a diluent, an emollient, a humectant, a moisturizer, an occlusive agent, oil, a penetration enhancer, a skin penetration enhancer, a perfuming agent, a pH adjusting agent, a preservative, a protectant, a softener, a solubilizer, a stearically stabilizing substance, a sun screening agent, a sunless tanning agent, a surfactant and a vitamin.

53. The spreadable pharmaceutical composition of claim 48, wherein a concentration of said Mupirocin and/or a Mupirocin derivative and/or a Mupirocin pharmaceutically acceptable salt ranges between about 0.5 weight percentage and about 5 weight percentages of the total weight of the composition.

54. The spreadable pharmaceutical composition of claim 51, wherein said pharmaceutically acceptable carrier comprises castor oil and/or oleyl alcohol.

55. The spreadable pharmaceutical composition of claim 48, further comprising at least one poly(alkylene)glycol (PAG).

56. The spreadable pharmaceutical composition of claim 55, wherein a concentration of said PAG is less than about 20 weight percentages of the total weight of the composition.

57. The spreadable pharmaceutical composition of claim 55, wherein a concentration of said PAG is less than about 10 weight percentages of the total weight of the composition.

58. The spreadable pharmaceutical composition of claim 55, wherein a concentration of said PAG is less than about 1 weight percentages of the total weight of the composition.

59. The spreadable pharmaceutical composition of claim 48, wherein a concentration of said at least one hard fat ranges between about 20 weight percentages and about 99 weight percentages of the total weight of the composition.

60. The spreadable pharmaceutical composition of claim 59, wherein a concentration of said at least one hard fat ranges between about 20 weight percentages and about 80 weight percentages of the total weight of the composition.

61. The spreadable pharmaceutical composition of claim 60, wherein said at least one hard fat comprises a triglyceride.

62. The spreadable pharmaceutical composition of claim 61, wherein said triglyceride is a mixed triester of glycerin with caprylic, capric and stearic acids.

63. The spreadable pharmaceutical composition of claim 52, wherein said additional component is a surfactant.

64. The spreadable pharmaceutical composition of claim 63, wherein said surfactant is propylene glycol stearate.

65. The spreadable pharmaceutical composition of claim 48, being substantially devoid of PAG.

66. The spreadable pharmaceutical composition of claim 48, having a viscosity that ranges between about 2 Poise and about 2500 Poise at a temperature of about 20 °C.

67. The spreadable pharmaceutical composition of claim 48, having a viscosity that ranges between about 2 Poise and about 1500 Poise at a temperature of about 20 °C.

68. The spreadable pharmaceutical composition of claim 48, being non-greasy, smooth and easy to spread.

69. The spreadable pharmaceutical composition of claim 54, comprising:
Mupirocin in a concentration of about 2 weight percentages;
at least one hard fat in a concentration of between about 20 weight percentages and about 80 weight percentages; and
a pharmaceutically acceptable carrier in a concentration of between about 20 weight percentages and about 80 weight percentages.

70. The spreadable pharmaceutical composition of claim 69, further comprising a surfactant in a concentration of between about 1 weight percentage and about 20 weight percentages.
